# EUROPEAN PATENT SPECIFICATION

(11) **EP 2 793 808 B1**
(45) Date of publication and mention of the grant of the patent: **17.05.2017**
(21) Application number: 11809026.5
(22) Date of filing: 20.12.2011
(51) Int. Cl.: A61K 8/25, A61K 8/34, A61K 8/73, A61Q 11/00

(54) **ORAL CARE COMPOSITIONS**
MUNDPFLEGEMITTEL
COMPOSITION DE SOINS BUCCAUX

(43) Date of publication of application: 29.10.2014
(73) Proprietor: Colgate-Palmolive Company, New York, NY 10022 (US)
(72) Inventor: FISHER, Steven, Middlesex, New Jersey 08846 (US); COLLIGAN, Mary, Hillsborough, New Jersey 08844 (US); PRENCIPE, Michael, Princeton Junction, New Jersey 08550 (US); TAMBS, Gary, Hillsborough, New Jersey 08844 (US)
(74) Representative: Wibbelmann, Jobst
(86) International application number: PCT/US2011/066093
(87) International publication number: WO 2013/095370

(56) References cited:
- WO-A1-01/32135
- WO-A1-98/58540
- US-A- 4 446 161
- US-A1- 2004 115 137
- US-A1- 2009 232 751
- US-B1- 6 673 384
- "Internation Cosmetic Ingredient Dictionary and Handbook", 2008, The Cosmetic, Toiletry, and Fragrance Association, XP002684675, vol. 1, pages 271-272, entry "BENZYL ALCOHOL", Functions

## Description

### BACKGROUND

Microbial contamination of oral care products poses a serious threat to the health of consumers. Thus, there is a need for oral care products that provide consistent and reproducible resistance to bacterial growth, while maintaining their efficacy and consumer acceptability.

Benzyl alcohol is known to be bacteriostatic and is used as a preservative for intravenous solutions. Benzyl alcohol is taught as being an effective preservative in high water content food products (US patent no. 4,446,161). Benzyl alcohol is also disclosed as being an ingredient in oral care compositions, but typically for its flavor properties (US patent no. 7,803,353). WO/2011/152819 teaches benzyl alcohol as part of a preservative system with other ingredients for use in an oral care composition; however, the water levels disclosed are no higher than 30%. WO 01/32135 discloses high water content dentifrice compositions and methods for making the same.

It would be highly desirable to have a new silica based oral care composition with ultra-high water content having adequate levels of microbiological robustness.

WO 2000003619 A1 discloses a toothbrush comprising a segmented, articulating head attached at one end to a handle, the segments being disposed longitudinally along the head and jointed to each other by an elastomer which is preferably fused to each segment.

DE 10221786 A1 discloses a toothbrush with a handle, to which a bristle bundles equipped flexible brush head is connected via a neck, characterized in that the brush head has an at least two-layer structure with at least one layer comprising a hard component and at least one layer comprising a soft component and that the bristle bundles with their fastening-side ends in a first layer forming component are arranged in and extend through the second layer.

US 20100263148 A1 disclose a toothbrush with a mechanical vibratory element and a head having a plurality of different types of cleaning areas which provide for an enhanced cleaning effect.

US 20110152909 A1 discloses an oral care implement with a soft tissue cleaner. The oral care implement comprises different kinds of soft tissue cleaners that offer expanded in-mouth cleaning.

### SUMMARY

The present inventors encountered significant challenges in preventing microbial growth and/or colonization during the manufacture and storage of silica based toothpastes containing greater than 50%, by weight, water. To overcome this challenge, the present inventors have discovered a preservative system suitable for use in silica based dentifrices, which provides optimal efficacy against microbial colonization and/or growth. Another challenge presented by ultra-high water compositions lies in the identification of an ingredient or combination of ingredients that provide an acceptable level of mouthfeel.

In one aspect, the present invention provides an oral care composition according to claim 1. In another aspect, the present invention provides a use according to claim 10. Also provided herein are oral care compositions comprising: greater than 50 weight % water; an effective amount of benzyl alcohol; and a silica abrasive. The present disclosure also provides methods of preventing microbial contamination of a toothpaste comprising greater than 50%, by weight, water. The present disclosure provides methods of preventing or reducing microbial colonization and/or growth in a silica based toothpaste comprising greater than 50%, by weight, water.

### DETAILED DESCRIPTION

As used herein, the term "oral composition" means the total composition that is delivered to the oral surfaces. The composition is further defined as a product which, during the normal course of usage, is not for the purposes of systemic administration of particular therapeutic agents or is not intentionally swallowed, but is rather retained in the oral cavity for a time sufficient to contact substantially all of the dental surfaces and/or oral tissues for the purposes of oral activity. Examples of such compositions include, but are not limited to, a toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

As used herein the term "effective amount" means an amount of benzyl alcohol effective to protect the composition against bacterial, yeast and mold growth.

As used herein "microbiological robustness" means the degree to which compositions are protected against bacterial, yeast and mold growth. The degree of microbiological robustness is indicated by the composition's MRT Value which can be ascertained by the methodology described in the Examples section herein. Compositions of the invention having adequate microbiological robustness have a MRT Value of 0.9 or greater.

As used herein "ultra-high water content" refers, but is not limited, to a toothpaste composition having a water concentration of 50% or greater. Thus, compositions of the invention typically have a water content of 50% to about 75%, in a particular embodiment about 55% to about 70%, in a more particular embodiment about 65%.

As noted above, a disadvantage of ultra-high water content oral compositions is inferior mouthfeel as perceived by consumers. It has been found that mouthfeel can be improved in such compositions by the addition of certain mouthfeel improving/enhancing agents. Thus, the present disclosure is further directed to a composition having improved mouthfeel comprising greater than about 50% water, a silica abrasive and an agent to improve mouthfeel, e.g., microcrystalline cellulose, maltodextrin, a modified food starch, or a combination thereof.

As used herein, the term "mouthfeel" relates to the perceived texture of the dentifrice observed by a user during brushing.

The present invention relates to an oral care composition according to claim 1.

In some embodiments, the mouthfeel enhancing agent is present at a concentration of from about 0.01% to about 5%, by weight, of the composition. In some embodiments, the mouthfeel enhancing agent is present at a concentration of from about 0.05% to about 2%, by weight, of the composition. In other embodiments, the mouthfeel enhancing agent is present at a concentration of about 1%, by weight, of the composition.

In some embodiments, the silica abrasive is present in the amount of from about 5% to about 20%, by weight, of the composition. In other embodiments, the silica abrasive is present at a concentration of about 8%, by weight, of the composition.

The benzyl alcohol is present at a concentration of from 0.2% to 0.6%, by weight, of the composition. In some embodiments, the benzyl alcohol is present at a concentration of from about 0.3% to about 0.5%, by weight, of the composition.

In some embodiments, the composition has a MRT Value greater than 0.9. In some embodiments, the composition has a MRT Value greater than 1.

The present disclosure provides for the use of benzyl alcohol, in a silica-based dentifrice, and a mouthfeel enhancing agent, in the preparation of an ultra-high water content oral care composition having microbiological robustness and acceptable mouthfeel.

A microcrystalline cellulose suitable for use in compositions of the present invention is the Avicel line of products, available from FMC Corporation. Suitable grades of Avicel include, but are not limited to, RC581, RC591, and CL611. In some embodiments, the compositions comprise Avicel RC591. In some embodiments, the microcrystalline cellulose has a rough porous surface, a spherical or non-spherical shape, a median particle diameter of about 20 to about 220 microns. In other embodiments, the microcrystalline cellulose has a tapped bulk density of from about 0.20 to about 0.45 grams/cubic centimeters.

Active and other ingredients useful herein may be categorized or described by their cosmetic and/or therapeutic benefit or their postulated mode of action. However, it is to be understood that the active and other ingredients useful herein can in some instances provide more than one cosmetic and/or therapeutic benefit or operate via more than one mechanism of action. Therefore, classifications are made for the sake of convenience and are not intended to limit an ingredient to the particularly stated application or the applications listed.

Some embodiments further comprise one or more pH modifying agents. In some embodiments, at least one of said one or more pH modifying agents is selected from the group consisting of: sodium hydroxide; potassium hydroxide; phosphoric acid; benzoic acid and citric acid.

In some embodiments, the pH of the composition is from about 7.8 to about 8.2.

The silica in the compositions of the invention can be in the form of silica gel, hydrated silica or precipitated silica. Examples of commercially available silica suitable for use in the invention are Zeodent 165 and Zeodent 114, available from J.M Huber Corporation. Some embodiments comprise other abrasives in addition to silica such, for example alumina (calcined or otherwise), bentonite, and insoluble phosphates such as tricalcium phosphate (Ca₃(PO₄)₂), hydroxyapatite (Ca₁₀(PO₄)₆(OH)₂), dicalcium phosphate dihydrate (CaHO₄ • 2H₂O, also sometimes referred to as DiCal), sodium metaphosphate, or calcium pyrophosphate. The abrasives also function as polishing agents. Other polishing agents optionally present in the compositions of the invention are sodium bicarbonate, calcium carbonate. Visually clear dentifrice compositions are obtained by using polishing agents such as collodial silica, such as those sold under the trade designation Zeodent 115 available from the J.M. Huber Corporation or alkali metal aluminosilicate complexes (that is, silica containing alumina combined in its matrix) which have refractive indices close to the refractive indices of gelling agent-liquid (including water and/or humectant) systems used in dentifrice compositions.

The silica abrasive polishing materials useful herein, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the above-noted Zeodent 114, 115 and 119. These silica abrasives are described in U.S. Pat. No. 4,340,583, to Wason. These silica abrasives are alkaline earth metal (e.g., calcium) treated silicone dioxides.

In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns.

In particular embodiments, the abrasive materials comprise a large fraction of very small particles, e.g., having a d50 < 5 microns, for example, small particle silica (SPS) having a d50 of about 3 to about 4 microns, for example Sorbosil AC43® (Ineos). Such small particles are particularly useful in formulations targeted at reducing hypersensitivity. The small particle component may be present in combination with a second larger particle abrasive. In certain embodiments, for example, the formulation comprises about 3 to about 8% SPS and about 25 to about 45% of a conventional abrasive.

Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention.

In some embodiments, the total amount of abrasive (i.e., silica and optionally other abrasives) is present in the oral care composition of the present disclosure at a concentration of from about 3% to about 60% by weight, in other embodiment about 5% to about 40% by weight, and in another embodiment about 7 to about 20% by weight. In some embodiments, the total amount of abrasive present in the composition is about 8%, by weight.

In some embodiments, compositions of the present invention further comprise safe and effective levels of one or more additional components. Such materials are well known and are readily chosen by those skilled in the art based on the oral care, physical and aesthetic properties desired for the compositions being prepared. Examples of such materials include, but are not limited to fats, solvents, waxes, emulsifiers, humectants, softeners, bulking agents, fluoride compounds, cationic materials, buffers, foaming agents, whitening agents, alkali metal bicarbonate salts, thickening agents, water, surfactants, anti-calculus agents, flavoring agents, sweeteners, coloring agents, breath fresheners, saliva stimulating agents, malodor control agents, amino acids, biomolecules, anti-inflammatory agents, antioxidants, vitamins, desensitizing agents, and mixtures thereof. It is specifically contemplated that one ingredient may provide two or more properties to the composition.

Some embodiments of the present invention provide compositions which further comprise a humectant. In some embodiments, the humectant is selected from the group consisting of: sorbitol; glycerin; polyethylene glycol; propylene glycol; and other edible polyhydric alcohols. Mixtures of humectants are commonly used, for example mixtures of sorbitol, glycerin and polyethylene glycol. In various embodiments, humectants are operable to prevent hardening of paste or gel compositions upon exposure to air. In some embodiments humectants also function as sweeteners.

Some embodiments comprise polyethylene glycol. In some embodiments, useful polyethylene glycols have a weight average molecular weight range of from about 200 to about 2000. A more particular embodiment is polyethylene glycols having a molecular weight range of from about 400 to about 1600 and another embodiment are polyethylene glycols having a molecular weight range of from about 570 to about 630.

The amount of humectant optionally present in the present invention is about 10% to about 20%, in some embodiments about 12% to about 15%, and in another embodiment about 14%, by weight, of the composition.

In some embodiments, the compositions of the present invention further comprise an amino acid. In some embodiments, the amino acid is present in a desensitizing effective amount. In some embodiments, the amino acid comprises from about 0.01% to about 20%, by weight, of the composition. In some embodiments, the amino acid comprises from about 1% to about 15%, by weight, of the composition. In some embodiments, the amino acid comprises from about 5% to about 10%, by weight, of the composition. In some embodiments, the amino acid comprises about 8%, by weight, of the composition. In some embodiments, the amino acid comprises about 4%, by weight, of the composition. In some embodiments, the amino acid comprises arginine. In some embodiments, the amino acid comprises L-arginine. In some embodiments, the amino acid comprises L-arginine bicarbonate.

In some embodiments the composition includes an anti-calculus agent. The anti-calculus agent is selected from: a phosphate, a pyrophosphate; a polyphosphate; a phosphonate; a polyphosphonate; and mixtures thereof. In some embodiments, the pyrophosphate is selected from: a dialkali metal pyrophosphate salt; a tetra-alkali metal pyrophosphate salt; and mixtures thereof in their unhydrated as well as hydrated forms. Disodium dihydrogen pyrophosphate (Na₂H₂P₂ O₇), tetrasodium pyrophosphate (Na₄P₂O₇) and tetrapotassium pyrophosphate (K₄P₂O₇) and mixtures thereof. Pyrophosphate salts suitable for use in the compositions of the present invention are described in more detail in Kirk and Othmer, Encyclopedia of Chemical Technology, 3rd Edition, Vol. 17, Wiley Interscience Publishers (1982).

Additional anti-calculus agents include polyacrylates and other polycarboxylates such as those disclosed in U.S. Pat. No. 3,429,963 and U.S. Pat. No. 4,304,766; and U.S. Pat. No. 4,661,341; polyepoxysuccinates such as those disclosed in U.S. Pat. No. 4,846,650; ethylenediaminetetraacetic acid as disclosed in British Patent No. 490,384; nitrilotriacetic acid and related compounds as disclosed in U.S. Pat. No. 3,678,154; polyphosphonates as disclosed in U.S. Pat. No. 3,737,533; U.S. Pat. No. 3,988,443 and U.S. Pat. No. 4,877,603. Anticalculus phosphates include potassium and sodium pyrophosphates; sodium tripolyphosphate; diphosphonates such as ethane-1-hydroxy-1, 1-diphosphonate, 1-azacycloheptane-1, 1-diphosphonate, and linear alkyl diphosphonates; linear carboxylic acids; and sodium zinc citrate and other soluble zinc salts.

The amount of anti-calculus agent optionally present in the composition of the invention is from about 0.1% to about 10%, by weight. In some embodiments, the anti-calculus agent is present in the amount of about 5% to about 8%, by weight.

Some embodiments further comprise a fluoride ion source. Examples of suitable fluoride ion sources can be found in U.S. Pat. Nos. 3,535,421 and 3,678,154. In some embodiments, the fluoride ion source is selected from: sodium fluoride; potassium fluoride; stannous fluoride; ammonium fluoride; sodium monofluorophosphate; and mixtures thereof. In a particular embodiment sodium fluoride is used. The amount of fluoride compound optionally present in the compositions of the invention is about 0.1% to about 1.5%, in some embodiments about 0.24%, in other embodiments about 0.454%, in further embodiments about 0.75%; and in other embodiments about 1.15%.

In some embodiments, the compositions of the present invention further comprise an oral malodor control agent. Such agents may include, but are not limited to, magnesium monopotassium phthalate; chlorhexidine; alexidine; hexetidine; sanguinarine; benzalkonium chloride; salicylanilide; domiphen bromide; cetylpyridinium chloride (CPC); tetradecylpyridinium chloride (TPC); N-tetradecyl-4-ethylpyridinium chloride (TDEPC); octenifine; delmopinol; octapinol; and other piperidine derivatives; nicin preparations; zinc/stannous ion agents; antibiotics such as augmentin, amoxicillin, tetracycline, doxycycline, minocycline, and metronidazole; and analogues and salts of the above; methyl salicyclate; and mixtures of all of the above.

Compositions of the present invention may also comprise surfactants, commonly referred to as sudsing agents. Suitable surfactants are those which are reasonably stable and foam throughout a wide pH range. The surfactant may be anionic, amphoteric, zwitterionic, cationic, or mixtures thereof.

Suitable anionic surfactants are disclosed in U.S. Pat. No. 3,959,458. In a particular embodiment sodium lauryl sulfate is the surfactant.

Nonionic surfactants which can be used in the compositions of the present invention can be broadly designed as compounds produced by the condensation of alkylene oxide groups (hydrophilic in nature) with an organic hydrophobic compound which may be aliphatic or alkylaromatic in nature.

The amphoteric surfactants useful in the present invention can be broadly described as derivatives of aliphatic secondary and tertiary amines in which the aliphatic radical can be straight chain or branched and wherein one of the aliphatic substituents contains froma bout 8 to about 18 carbon atons and one contains an anionic water solubilising group e.g., carboxylate, sulphonate, suphate, phosphate or phosphonate. Many of these suitable non-ionic and amphoteric surfactants are disclosed in U.S. Pat. No. 4,051,234.

The amount of surfactant optionally present in the composition of the invention is about 0.1% to about 5%, in some embodiments about 1% to about 3%, in a other embodiments about 1.5%, by weight.

Other optional additives include antimicrobial (e.g., antibacterial) agents. Any orally acceptable antimicrobial agent can be used, including halogentated diphenylethers such as triclosan (5-chloro-2-(2,4-dichlorophenoxy)phenol); 8-hydroxyquinoline and salts thereof, zinc and stannous ion sources such as zinc citrate, zinc sulphate, zinc glycinate, sodium zinc citrate and stannous pyrophosphate; copper (II) compounds such as copper (II) chloride, fluoride, sulfate and hydroxide; phthalic acid and salts thereof such as magnesium monopotassium phthalate; sanguinarine; quaternary ammonium compounds, such as alkylpyridinium chlorides (e.g., cetylpyridinium chloride (CPC), combinations of CPC with zinc and/or enzymes, tetradecylpyridinium chloride, and N-tetradecyl-4-ethylpyridinium chloride,); bisguanides, such as chlorhexidine digluconate, hexetidine, octenidine, alexidine; halogenated bisphenolic compounds, such as 2,2' methylenebis-(4-chloro-6-bromophenol); benzalkonium chloride; salicylanilide, domiphen bromide; iodine; sulfonamides; bisbiguanides; phenolics; piperidino derivatives such as delmopinol and octapinol; magnolia extract; grapeseed extract; thymol; eugenol; menthol; geraniol; carvacrol; citral; eucalyptol; catechol; 4-allylcatechol; hexyl resorcinol; methyl salicylate; and mixtures thereof. A further illustrative list of useful antibacterial agents is provided in U.S. Pat. No. 5,776,435.

Antioxidants are another class of optional additives. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

Also optional, a saliva stimulating agent, useful for example in amelioration of dry mouth, may be included. Any orally acceptable saliva stimulating agent can be used, including without limitation food acids such as citric, lactic, malic, succinic, ascorbic, adipic, fumaric, and tartaric acids, and mixtures thereof. One or more saliva stimulating agents are optionally present in a saliva stimulating effective amount.

Optional breath freshening agents may be provided. Any orally acceptable breath freshening agent can be used, including without limitation zinc salts such as zinc gluconate, zinc citrate and zinc chlorite, alpha-ionone and mixtures thereof. One or more breath freshening agents are optionally present in a breath freshening effective total amount.

Another optional ingredient in the composition of the invention is a thickening agent to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as xanthan gum, karaya, gum arabic, and gum tragacanth can also be incorporated. Colloidal magnesium aluminum silicate or finely divided silica can be used as component of the thickening composition to further improve the composition's texture. In an embodiment carboxymethylcellulose, xanthan gum, or a mixture thereof is used. In certain embodiments, thickening agents in an amount of about 0.1% to about 10.0% are used.

Flavoring agents for incorporation in the compositions may include natural and artificial flavors. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavor agents can be used individually or in admixture. Commonly used flavors include mints such as peppermint, artificial vanilla, cinnamon derivatives, and various fruit flavors, whether employed individually or in admixture. Generally, any flavoring or food additive, such as those described in Chemicals Used in Food Processing, publication 1274 by the National Academy of Sciences, pages 63-258, may be used.

The amount of flavoring agent optionally present in the compositions of the invention is about 0.5% to about 2%, in some embodiments about 0.08% to about 1.2%, and in other embodiments, about 1%, by weight.

The compositions of the invention optionally contain a sweetening agent. Sweetening agents which can be used include sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, in particular sucralose, sodium cyclamate and sodium saccharin, and mixtures thereof. In an embodiment sodium saccharin is the sweetener. A composition may contain from about 0.1% to about 10% of a sweetener, preferably from about 0.1% to about 1%, by weight of the total composition, in some embodiments about 0.3%, and in a particular embodiment 0.27%, by weight.

The compositions of the present invention may also comprise colorants. In some embodiments, the colorant can be a dye or a pigment. Dyes suitable for use in compositions of the present invention may be food color additives presently certified under the Food Drug & Cosmetic Act for use in food and ingested drugs, including dyes such as FD&C Red No. 3 (sodium salt of tetraiodofluorescein), Food Red 17, disodium salt of 6-hydroxy-5-{(2-methoxy-5-methyl-4-sulphophenyl)azo}-2-n- aphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sul- fophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2- -sulfoniumphenyl)-methylene}-[1-(N-ethyl-N-p-sulfobenzyl)-.DELTA.-3,5-cycl- ohexadienimine], FD&C Blue No. 1 (disodium salt of dibenzyldiethyl-diamino-triphenylcarbinol trisulfonic acid anhydrite), FD&C Blue No. 2 (sodium salt of disulfonic acid of indigotin) and mixtures thereof in various proportions. In an embodiment titanium dioxide is the colorant. The amount of colorant optionally present in the composition of the invention is about 0.001% to about 1%, in some embodiments about 0.5%, by weight.

In addition, compositions of the invention optionally comprise one or more of the following ingredients: antimicrobial agents other than benzyl alcohol, e.g., triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bisguanides, such as chlorhexidine digluconate, hexetidine, octenidine, alexidine; and halogenated bisphenolic compounds, such as 2,2' methylenebis-(4-chloro-6-- bromophenol); antiinflammatory agents such as ibuprofen, flurbiprofen, aspirin, indomethacine; plaque buffers such as urea, calcium lactate, calcium glycerophosphate and strontium polyacrylates; vitamins such as Vitamin C; plant extracts; desensitizing agents, e.g., potassium citrate, potassium chloride, potassium tartrate, potassium bicarbonate, potassium oxalate, potassium nitrate and strontium salts; biomolecules, e.g., bacteriocins, antibodies, enzymes such as papain, glucoamylase; and opacifying agents.

Some embodiments provide a composition obtained or obtainable by combining the ingredients as set forth in any of the preceding compositions.

Some embodiments provide a composition in the form of a paste or a gel.

Some embodiments of the present disclosure provide methods of inhibiting a disease, disorder or condition of the oral cavity comprising contacting an oral cavity surface with any of the compositions described herein. Some embodiments of the present disclosure provide methods of preventing a disease, disorder or condition of the oral cavity comprising contacting an oral cavity surface with any of the compositions described herein. Some embodiments of the present disclosure provide methods of treating a disease, disorder or condition of the oral cavity comprising contacting an oral cavity surface with any of the compositions described herein. In some embodiments, the disease, disorder or condition of the oral cavity is an inflammatory disease, disorder or condition. In some embodiments, the disease, disorder or condition of the oral cavity is selected from the group consisting of: gingivitis; periodontitis; and halitosis. In some embodiments, the present disclosure provides methods of whitening a tooth surface comprising contacting a tooth surface with any of the compositions described herein.

In another embodiment the disclosure includes use of benzyl alcohol, a silica abrasive, and a mouthfeel enhancing agent selected from microcrystalline cellulose, maltodextrin or a mixture thereof, to prepare a high water content oral care composition according to claim 1.

The compositions of the present invention can be made using methods which are common in the oral product area.

All percentages in this specification and claims are by weight of the total composition, unless specifically stated otherwise.

As used throughout, ranges are used as shorthand for describing each and every value that is within the range. Any value within the range can be selected as the terminus of the range.

In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

### EXAMPLES

### Reference Example 1

Table 1 (below) provides the formulations for two exemplary compositions of the present disclosure (Formulae I and II) and four compositions which are similarly formulated, but do not contain the inventive combination of the present disclosure.

**Table 1**

| **Ingredient** | **I** | **II** | **CI** | **CII** | **CIII** | **CIV** |
|---|---|---|---|---|---|---|
| Deionized water | 64.79 | 64.59 | 65.09 | 64.59 | 64.59 | 64.29 |
| Glycerin | 13.2 | 13.2 | 13.2 | 13.2 | 13.2 | 13.2 |
| PEG-600 | 1 | 1 | 1 | 1 | 1 | 1 |
| CMC | 0.3 | 0.3 | 0.3 | 0.3 | 0.3 | 0.3 |
| Xanthan gum | 0.2 | 0.2 | 0.2 | 0.2 | 0.2 | 0.2 |
| Saccharin | 0.27 | 0.27 | 0.27 | 0.27 | 0.27 | 0.27 |
| Sodium pyrophosphate | 0.5 | 0.5 | 0.5 | 0.5 | 0.5 | 0.5 |
| Sodium fluoride | 0.24 | 0.24 | 0.24 | 0.24 | 0.24 | 0.24 |
| Zeodent 165 | 8 | 8 | 8 | 8 | 8 | 8 |
| Zeodent 114 | 8 | 8 | 8 | 8 | 8 | 8 |
| Flavorant | 1.2 | 1.2 | 1.2 | 1.2 | 1.2 | 1.2 |
| Titanium dioxide | 0.5 | 0.5 | 0.5 | 0.5 | 0.5 | 0.5 |
| Sodium lauryl sulfate | 1.5 | 1.5 | 1.5 | 1.5 | 1.5 | 1.5 |
| Benzyl alcohol | 0.3 | 0.5 | -- | -- | -- | -- |
| Microcrystalline cellulose | -- | -- | -- | -- | -- | -- |
| Maltodextrin | -- | -- | -- | -- | -- | -- |
| Potassium sorbate | -- | -- | -- | -- | -- | 0.8 |
| Sodium benzoate | -- | -- | -- | 0.5 | -- | -- |
| Cetylpyridinium chloride | -- | -- | -- | -- | 0.5 | -- |
| Maltodextrin | -- | -- | -- | -- | -- | -- |
| Potassium sorbate | -- | -- | -- | -- | -- | 0.8 |
| Sodium benzoate | -- | -- | -- | 0.5 | -- | -- |
| Cetylpyridinium chloride | -- | -- | -- | -- | 0.5 | -- |

### Reference Example 2

Disperse carboxymethyl cellulose, xanthan gum and microcrystalline cellulose in formula weight of glycerin and add to main mixing vessel that contains 85% of formula weight of water. Solubilize sodium saccharin and sodium fluoride in remaining water and add to main mixing beaker.

Disperse tetrasodium pyrophosphate in formula weight of polyethylene glycol and add to main mixing beaker and mix for 20 minutes to complete the gel.

Transfer gel to a suitable mixer and charge silicas and complete 20 minute silica mix. Mix benzyl alcohol in flavor oil. Add flavor mix and sodium lauryl sulfate to silca mix and mix for about 15 minutes until homogenous composition is obtained.

### Reference Example 3

Compositions of the present disclosure are compared to the similarly formulated products without benzyl alcohol for microbiological robustness. This test is a quantitative measure of the formula's ability to withstand microbial insult, both at the plant and in the hands of the consumers, and encompasses the rate of kill of the bacterial inoculum as well as the total kill level. This quantitative measure is defined as the MRT value.

Compositions are challenged with a selected inoculum pool. At selected time intervals, the inoculated test material is sampled. Dilutions and platings are performed to recover the surviving organisms. The log differences in the bacterial count (Log₁₀ reduction) between the product and the inoculum control is calculated over time to determine the MRT value. The results indicate the effectiveness of a preservative or bacteriostatic system - the greater the MRT value, the more effective the preservative. An MRT value of greater than 0.9 is desired for optimal effectiveness.

**Table 2**

| **Composition** | **MRT Value** |
|---|---|
| Formula I | 1.03 |
| Formula II | 1.1 |
| CI | 0.57 |
| CII | 0.82 |
| CIII | 0.51 |
| CIV | 0.75 |

Table 2 (above) describes data generated from a micro-robustness evaluation of compositions of the present invention versus comparative examples without benzyl alcohol. The data described therein, demonstrates that compositions of the present invention provide an unexpected level of resistance to microbial contamination.

## Claims

1. An oral care composition comprising:
from 50% to 80%, by weight, water;
from 0.2 to 0.6 %, by weight, benzyl alcohol; and
from 3 to 25%, by weight of a silica abrasive; and further comprising a mouthfeel enhancing agent selected from: microcrystalline cellulose; maltodextrin; and a combination thereof, in an amount effective to enhance the mouthfeel of the composition during use.

2. The composition of claim 1, wherein the oral care composition is selected from the group consisting of toothpaste, dentifrice, mouthwash, mouthrinse, topical oral gel and denture cleanser.

3. The composition of claim 1 or claim 2, wherein the mouthfeel enhancing agent comprises microcrystalline cellulose.

4. The composition of any preceding claim, wherein the mouthfeel enhancing agent is present at a concentration of from 0.01% to 5%, by weight, of the composition.

5. The composition of claim 4, wherein the mouthfeel enhancing agent is present at a concentration of from 0.05% to 2%, by weight, of the composition.

6. The composition of claim 5, wherein the mouthfeel enhancing agent is present at a concentration of 1%, by weight, of the composition.

7. The composition of any foregoing claim, wherein the silica abrasive is present in an amount of from 5% to 20%, by weight, of the composition.

8. The composition of claim 7, wherein the silica abrasive is present at a concentration of 8%, by weight, of the composition.

9. The composition of any foregoing claim, wherein the benzyl alcohol is present at a concentration of from 0.3% to 0.5%, by weight, of the composition.

10. Use of benzyl alcohol, a silica abrasive agent, and a mouthfeel enhancing agent selected from microcrystalline cellulose, maltodextrin or a mixture thereof, to prepare an oral care composition according to claim 1.

## Patentansprüche

1. Mundpflege-Zusammensetzung, umfassend:
von 50% bis 80%, bezogen auf das Gewicht, an Wasser;
von 0,2 bis 0,6%, bezogen auf das Gewicht, an Benzylalkohol; und
von 3 bis 25%, bezogen auf das Gewicht, an Silica-Abrasivstoff; und weiterhin umfassend ein das Mundgefühl-verbesserndes Mittel, ausgewählt aus: mikrokristalliner Cellulose; Maltodextrin; und einer Kombination davon, in einer Menge, die wirksam ist, um das Mundgefühl der Zusammensetzung während der Verwendung zu verbessern.

2. Die Zusammensetzung nach Anspruch 1, wobei die Mundpflege-Zusammensetzung ausgewählt ist aus der Gruppe, bestehend aus Zahnpasta, Zahnreinigungsmittel, Mundwasser, Mundspülung, topisches Mundgel und Gebissreiniger.

3. Die Zusammensetzung nach Anspruch 1 oder Anspruch 2, wobei das das Mundgefühl-verbessernde Mittel mikrokristalline Cellulose umfasst.

4. Die Zusammensetzung nach einem beliebigen vorangegangenen Anspruch, wobei das das Mundgefühl-verbessernde Mittel in einer Konzentration von 0,01% bis 5%, bezogen auf das Gewicht der Zusammensetzung, vorliegt.

5. Die Zusammensetzung nach Anspruch 4, wobei das das Mundgefühlverbessernde Mittel in einer Konzentration von 0,05% bis 2%, bezogen auf das Gewicht der Zusammensetzung, vorliegt.

6. Die Zusammensetzung nach Anspruch 5, wobei das das Mundgefühlverbessernde Mittel in einer Konzentration von 1%, bezogen auf das Gewicht der Zusammensetzung, vorliegt.

7. Die Zusammensetzung nach einem beliebigen vorangegangenen Anspruch, wobei der Silica-Abrasivstoff in einer Menge von 5% bis 20%, bezogen auf das Gewicht der Zusammensetzung, vorliegt.

8. Die Zusammensetzung nach Anspruch 7, wobei der abrasive Silica-Stoff in einer Konzentration von 8%, bezogen auf das Gewicht der Zusammensetzung, vorliegt.

9. Die Zusammensetzung nach einem beliebigen vorangegangenen Anspruch, wobei der Benzylalkohol in einer Konzentration von 0,3% bis 0,5%, bezogen auf das Gewicht der Zusammensetzung, vorliegt.

10. Verwendung von Benzylalkohol, einem Silica- Abrasivmittel und einem Mundgefühl-verbessernden Mittel, ausgewählt aus mikrokristalliner Cellulose, Maltodextrin oder einer Mischung davon, um eine Mundpflege-Zusammensetzung gemäß Anspruch 1 herzustellen.

## Revendications

1. Composition de soin buccal comprenant :
de 50 % à 80 % en poids d'eau ;
de 0,2 à 0,6 % en poids d'alcool benzylique ; et
de 3 à 25 % en poids d'un abrasif à base de silice ; et comprenant en outre un agent améliorant la sensation en bouche choisi parmi : la cellulose microcristalline ; la maltodextrine ; et une combinaison de celles-ci, en une quantité efficace pour améliorer la sensation en bouche de la composition pendant l'utilisation.

2. Composition selon la revendication 1, dans laquelle la composition de soin buccal est choisie dans le groupe constitué par une pâte dentifrice, un dentifrice, un bain de bouche, un rince-bouche, un gel topique oral et un nettoyant pour prothèse dentaire.

3. Composition selon la revendication 1 ou la revendication 2, dans laquelle l'agent améliorant la sensation en bouche comprend de la cellulose microcristalline.

4. Composition selon l'une quelconque des revendications précédentes, dans laquelle l'agent améliorant la sensation en bouche est présent à une concentration de 0,01 % à 5 % en poids de la composition.

5. Composition selon la revendication 4, dans laquelle l'agent améliorant la sensation en bouche est présent à une concentration de 0,05 % à 2 % en poids de la composition.

6. Composition selon la revendication 5, dans laquelle l'agent améliorant la sensation en bouche est présent à une concentration de 1 % en poids de la composition.

7. Composition selon l'une quelconque des revendications précédentes, dans laquelle l'abrasif à base de silice est présent en une quantité de 5 % à 20 % en poids de la composition.

8. Composition selon la revendication 7, dans laquelle l'abrasif à base de silice est présent à une concentration de 8 % en poids de la composition.

9. Composition selon l'une quelconque des revendications précédentes, dans laquelle l'alcool benzylique est présent à une concentration de 0,3 % à 0,5 % en poids de la composition.

10. Utilisation d'alcool benzylique, d'un agent abrasif à base de silice et d'un agent améliorant la sensation en bouche choisi parmi la cellulose microcristalline, la maltodextrine ou un mélange de celles-ci, pour préparer une composition de soin buccal selon la revendication 1.
